(19)

Europäisches
Patentamt
European
Patent Office
Office européen
des brevets

(11)  **EP 2 781 260 A1**

(12)  # EUROPEAN PATENT APPLICATION
published in accordance with Art. 153(4) EPC

(43) Date of publication:
**24.09.2014 Bulletin 2014/39**

(21) Application number: **12849609.8**

(22) Date of filing: **16.11.2012**

(51) Int Cl.:
**B01J 27/199** [(2006.01)]  **C07C 51/235** [(2006.01)]
**C07C 57/055** [(2006.01)]  **C07B 61/00** [(2006.01)]

(86) International application number:
**PCT/JP2012/079862**

(87) International publication number:
**WO 2013/073691 (23.05.2013 Gazette 2013/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2011 JP 2011251386**

(71) Applicant: **Nippon Kayaku Kabushiki Kaisha Tokyo 102-8172 (JP)**

(72) Inventors:
• **IIJIMA,Takayuki**
**Akishima-shi**
**Tokyo 196-0013 (JP)**

• **KURAKAMI, Tatsuhiko**
**Sanyoonoda-shi**
**Yamaguchi 757-8686 (JP)**
• **NISHIMURA, Eiji**
**Sanyoonoda-shi**
**Yamaguchi 757-8686 (JP)**
• **EJIRI, Tomoyuki**
**Sanyoonoda-shi**
**Yamaguchi 757-8686 (JP)**
• **SAKAI, Hideomi**
**Sanyoonoda-shi**
**Yamaguchi 757-8686 (JP)**

(74) Representative: **Gille Hrabal**
**Brucknerstrasse 20**
**40593 Düsseldorf (DE)**

(54)  **CATALYST FOR PRODUCTION OF METHACRYLIC ACID AND METHOD FOR PRODUCING METHACRYLIC ACID USING SAME**

(57)  There is provided a hetero polyacid-based catalyst for methacrylic acid production, which is more excellent in performance, life and moisture absorption during storage. A catalyst for methacrylic acid production, wherein a proton is replaced so as to satisfy conditions of $\alpha = A + (B \times C)$ and $0.5 \leq \alpha \leq 1.4$ when the atomic ratio of the alkali metal atom relative to 10 atoms of Mo is taken as A and the atomic ratio of the copper atom relative to 10 atoms of Mo is taken as B in $Mo_aP_bV_cCu_dY_eZ_fO_g$ where Y represents cesium or the like; Z represents iron or the like; and a to g represent each an atomic ratio of each element relative to 10 atoms of Mo.

EP 2 781 260 A1

**Description**

Technical Field

The present invention relates to a hetero polyacid-based catalyst used in the production of methacrylic acid by vapor-phase catalytic oxidation of at least one selected from the group consisting of methacrolein, isobutyraldehyde and isobutyric acid with a molecular oxygen-containing gas in the presence of an oxidation catalyst composition and to a process for producing methacrylic acid using the same.

Background Art

Hitherto, as catalysts used in the production of methacrylic acid by vapor-phase catalytic oxidation of methacrolein or the like, it is known that those composed of a hetero polyacid and/or a salt thereof are effective, and there are a large number of reports regarding compositions, structures, physical properties, and production processes thereof (for example, Patent Document 1 describes pores of catalysts, Patent Document 2 describes a shaping method in catalyst production, and Patent Document 3 describes a calcination method in catalyst production).
A hetero polyacid-based catalyst used in the production of methacrylic acid by vapor-phase catalytic oxidation of at least one selected from the group consisting of methacrolein, isobutyraldehyde and isobutyric acid with a molecular oxygen-containing gas in the presence of an oxidation catalyst composition has a high moisture absorbability and, once it absorbs moisture, it is known that it is difficult to enhance activity again although the difficultness depends on the degree of moisture absorption. Thus, there is a problem that catalytic performance decreases owing to the moisture absorption during the storage from the production of the catalyst until the use thereof and satisfactory conversion and selectivity of methacrylic acid are not obtained in methacrylic acid production.
Patent Document 4 describes an invention that a catalyst for methacrylic acid production is stored in a container having a moisture permeability of 1.0 $g/m^2 \cdot 24h$ or less at 25°C. Patent Document 5 describes an invention that air is introduced so that relative humidity in a catalyst layer is controlled to 40% or less to prevent moisture absorption of a catalyst. However, these known technologies are useful as methods for preventing moisture absorption of the catalyst for methacrylic acid production but there is a problem that any of steps from the production of the catalyst until the use thereof becomes tedious and complex.
On the other hand, the hetero polyacid-based catalyst used in the production of methacrylic acid from at least one selected from the group consisting of methacrolein, isobutyraldehyde and isobutyric acid is indeed inferior in life to the oxidation catalyst for producing acrylic acid from acrolein.
Patent Document 6 discloses a process for producing a catalyst for methacrylic acid production by mixing a homogeneous solution containing a fourth ingredient X such as molybdenum, vanadium, phosphorus and antimony with a homogeneous solution containing the other catalyst ingredients such as ammonia water and cesium and drying the mixed solution. Thereby, it is said that solubility of the fourth ingredient X (especially antimony) is enhanced and a catalyst exhibiting excellent reproducibility and stability of catalytic performance and having a long life is obtained. Moreover, Patent Document 7 discloses a production process of an oxidation catalyst wherein, with regard to a solution obtained by dissolving or suspending all catalyst raw materials in water, the content of an ammonium radical is controlled to the range of 17 to 100 mol relative to 12 atoms of molybdenum and pH thereof is controlled to the range of 6.5 to 13. The control of the pH is performed through addition of nitric acid, ammonia water, or the like.
However, catalysts produced using such conventional method for mixing catalyst raw materials or method for controlling pH are not always sufficient as industrial catalysts particularly in view of life and it is desired to further improve catalytic performance.

Background Art Document

Patent Document

Patent Document 1: JP-A-60-239439
Patent Document 2: JP-A-10-258233
Patent Document 3: JP-A-59-66349
Patent Document 4: JP-A-2003-10695
Patent Document 5: Japanese Patent No. 3884967
Patent Document 6: JP-A-5-31368
Patent Document 7: JP-A-9-290162

Summary of Invention

Problem that Invention is to Solve

An object of the present invention is to provide a hetero polyacid-based catalyst, which is more excellent in performance, life, and also moisture absorption during storage that is particularly important at industrial use, as compared with hetero polyacid-based catalysts used in the production of methacrylic acid by vapor-phase catalytic oxidation of methacrolein with molecular oxygen and a process for producing methacrylic acid using the same.

Means for Solving Problem

As a result of extensive studies on hetero polyacid-based catalysts used in the production of methacrylic acid by vapor-phase catalytic oxidation of at least one selected from the group consisting of methacrolein, isobutyraldehyde and isobutyric acid with a molecular oxygen-containing gas in the presence of an oxidation catalyst composition for solving the above problems, the present inventors have found that the above problems can be solved by a hetero polyacid-based catalyst used for producing methacrylic acid by vapor-phase catalytic oxidation of methacrolein with molecular oxygen, wherein the catalyst has a composition represented by the following general formula:

$$Mo_aP_bV_cCu_dY_eZ_fO_g$$

wherein Mo, P, V, Cu and O represent molybdenum, phosphorus, vanadium, copper and oxygen, respectively; Y represents at least one element selected from potassium, rubidium, cesium and thallium; Z represents at least one element selected from iron, cobalt, zinc, chromium, magnesium, tantalum, manganese, gallium, barium, cerium, lanthanum, arsenic, antimony, bismuth, germanium, ammonium, zirconium, tin, lead, titanium, tellurium, silver, selenium, silicon, tungsten and boron; a, b, c, d, e, f and g represent each an atomic ratio of each element and, when a is 10, b is 0.1 or more and 4 or less, c is 0.01 or more and 4 or less, d is 0.01 or more and 1 or less, e is 0.2 or more and 2 or less, f is 0 or more and 3 or less, and g is a numerical value determined depending on oxidation states of individual elements, and conditions:

$$\alpha = A + (B \times C)$$

$$0.5 \leq \alpha \leq 1.4$$

are satisfied when the atomic ratio of the alkali metal atom relative to 10 atoms of molybdenum in the hetero polyacid and hetero polyacid salt containing molybdenum, phosphorus, vanadium and copper as essential ingredients is taken as A and the atomic ratio of the copper atom relative to 10 atoms of molybdenum in the hetero polyacid and hetero polyacid salt is taken as B, and the valence number is taken as C. In this regard, with regard to the valence number of the copper atom, the valence number in a raw material used is maintained as it is in the catalyst. Thus, they have accomplished the present invention based on the findings.

Namely, the invention relates to:

(1) A catalyst for methacrylic acid production used for producing methacrylic acid by vapor-phase catalytic oxidation of methacrolein with molecular oxygen,
the catalyst having a composition represented by the following general formula: $MO_aP_bV_cCu_dY_eZ_fO_g$
wherein Mo, P, V, Cu, and O represent molybdenum, phosphorus, vanadium, copper and oxygen, respectively; Y represents at least one element selected from potassium, rubidium, cesium and thallium; Z represents at least one element selected from iron, cobalt, zinc, chromium, magnesium, tantalum, manganese, gallium, barium, cerium, lanthanum, arsenic, antimony, bismuth, germanium, ammonium, zirconium, tin, lead, titanium, tellurium, silver, selenium, silicon, tungsten and boron; a, b, c, d, e, f and g represent each an atomic ratio of each element and, when a is 10, b is 0.1 or more and 4 or less, c is 0.01 or more and 4 or less, d is 0.01 or more and 1 or less, e is 0.2 or more and 2 or less, f is 0 or more and 3 or less, and g is a numerical value determined depending on oxidation states of individual elements,
wherein a proton that is a counter cation is replaced by an alkali metal ion so as to satisfy conditions:

EP 2 781 260 A1

$$\alpha = A + (B \times C)$$

$$0.5 \leq \alpha \leq 1.4$$

when an atomic ratio of the alkali metal atom relative to molybdenum atom in a hetero polyacid and hetero polyacid salt containing molybdenum, phosphorus, vanadium and copper as essential ingredients is taken as A and an atomic ratio of copper atom relative to molybdenum atom in the hetero polyacid and hetero polyacid salt is taken as B, and a valence number is taken as C;

(2) The catalyst for methacrylic acid production according to (1), which satisfies a condition:

$$0.7 \leq \alpha \leq 1.1;$$

(3) The catalyst for methacrylic acid production according to (1) or (2),
wherein Y is cesium;
(4) The catalyst for methacrylic acid production according to any one of (1) to (3), which satisfies conditions that d is 0.1 or more and 0.3 or less and e is 0.3 or more and 1.1 or less when a is 10;
(5) The catalyst for methacrylic acid production according to any one of (1) to (4), which satisfies conditions that d is 0.15 or more and 0.25 or less and e is 0.4 or more and 1.0 or less when a is 10;
(6) The catalyst for methacrylic acid production according to any one of (1) to (5),
wherein the catalyst is a shaped catalyst;
(7) A process for producing methacrylic acid by vapor-phase catalytic oxidation of methacrolein, isobutyraldehyde and isobutyric acid, the process comprising using the catalyst according to any one of (1) to (6).

Advantage of the Invention

According to the present invention, it becomes possible to provide a hetero polyacid-based catalyst used in the production of methacrylic acid by vapor-phase catalytic oxidation of at least one selected from the group consisting of methacrolein, isobutyraldehyde and isobutyric acid, which is more excellent in performance, life and also moisture absorption during storage that is particularly important at industrial use.

Mode for Carrying Out the Invention

The following will describe the present invention in detail but the range of the invention to be applied should not be construed as being limited to the following methods. A catalyst for methacrylic acid production used for producing methacrylic acid by vapor-phase catalytic oxidation of methacrolein with molecular oxygen according to the invention is characterized in that the catalyst has a composition represented by the following general formula:

$$Mo_a P_b V_c Cu_d Y_e Z_f O_g$$

wherein Mo, P, V, Cu and O represent molybdenum, phosphorus, vanadium, copper and oxygen, respectively; Y represents at least one element selected from potassium, rubidium, cesium and thallium; Z represents at least one element selected from iron, cobalt, zinc, chromium, magnesium, tantalum, manganese, gallium, barium, cerium, lanthanum, arsenic, antimony, bismuth, germanium, ammonium, zirconium, tin, lead, titanium, tellurium, silver, selenium, silicon, tungsten and boron; a, b, c, d, e, f and g represent each an atomic ratio of each element and, when a is 10, b is 0.1 or more and 4 or less, c is 0.01 or more and 4 or less, d is 0.01 or more and 1 or less, e is 0.2 or more and 2 or less, f is 0 or more and 3 or less, and g is a numerical value determined depending on oxidation states of individual elements, and a proton that is a counter cation is replaced by an alkali metal ion so as to satisfy conditions:

$$\alpha = A + (B \times C)$$

$$0.5 \leq \alpha \leq 1.4$$

4

when the atomic ratio of the alkali metal atom relative to 10 atoms of molybdenum in the hetero polyacid and hetero polyacid salt containing molybdenum, phosphorus, vanadium and copper as essential ingredients is taken as A and the atomic ratio of the copper atom relative to 10 atoms of molybdenum in the hetero polyacid and hetero polyacid salt is taken as B, and the valence number is taken as C. In this regard, with regard to the valence number of the copper atom, the valence number in a raw material used is maintained as it is in the catalyst.

Here, the proton of the hetero polyacid is an important chemical structure for an attracting effect of methacrolein molecule and for oxidation into methacrylic acid through supply of oxygen of the hetero polyacid. On the other hand, the hetero polyacid salt has a weak attracting effect of methacrolein molecule since the proton is decreased and thus reactivity of methacrolein decreases. However, the hetero polyacid salt has an effect of suppressing a sequential oxidation reaction. That is, the salt suppress the sequential oxidation reaction where, after methacrolein is oxidized into methacrylic acid, an oxidation reaction further continues to result in combustion and hence carbon monoxide, carbon dioxide, acetic acid, and the like are produced as by-products. By the effect, selectivity of methacrylic acid can be improved.

As a further preferable catalyst as the catalyst, there is suitably used a catalyst for methacrylic acid production represented by the following general formula:

$$Mo_aP_bV_cCu_dY_eZ_fO_g$$

wherein Mo, P, V, Cu and O represent molybdenum, phosphorus, vanadium, copper and oxygen, respectively; Y represents at least one element selected from potassium, rubidium, cesium and thallium; Z represents at least one element selected from iron, cobalt, zinc, chromium, magnesium, tantalum, manganese, gallium, barium, cerium, lanthanum, arsenic, antimony, bismuth, germanium, ammonium, zirconium, tin, lead, titanium, tellurium, silver, selenium, silicon, tungsten and boron; a, b, c, d, e, f and g represent each an atomic ratio of each element and, when a is 10, b is 0.1 or more and 4 or less, c is 0.01 or more and 4 or less, d is 0.01 or more and 1 or less, e is 0.2 or more and 2 or less, f is 0 or more and 3 or less, and g is a numeric value determined depending on oxidation states of individual elements.

$\alpha$ is calculated by an expression:

$$\alpha = A + (B \times C)$$

when the atomic ratio of the alkali metal atom relative to 10 atoms of molybdenum in the hetero polyacid and hetero polyacid salt is taken as A and the atomic ratio of the copper atom relative to 10 atoms of molybdenum in the hetero polyacid and hetero polyacid salt is taken as B, and the valence number is taken as C. In this regard, with regard to the valence number of the copper atom, the valence number in a raw material used is maintained as it is in the catalyst In the catalyst, when a = 10, $0.5 \leq \alpha$ 1.4, preferably $0.7 \leq \alpha \leq 1.1$. When $\alpha$ is smaller than 0.5, there is a case where activity of the resulting catalyst decreases. On the other hand, when $\alpha$ is larger than 1.7, the life of the resulting catalyst tends to decrease.

In the catalyst, when a = 10, $0.2 \leq e \leq 2.0$, preferably $0.5 \leq e \leq 1.0$, and more preferably $0.6 \leq e \leq 0.9$. When e is smaller than 0.2, moisture absorbability of the resulting catalyst is high and catalytic performance decreases during the period from catalyst production until its use, so that satisfactory catalytic performance is sometimes not obtained at methacrylic acid production. On the other hand, when e is larger than 2.0, there is a case where the life of the resulting catalyst decreases.

The catalyst production process of the invention comprises a step of preparing an aqueous solution containing a compound containing catalyst active ingredients (molybdenum, phosphorus, vanadium and copper; hereinafter referred to as essential ingredients) or a water dispersion of the compound (hereinafter collectively referred to as a slurry), a step of drying the slurry and sometimes calcining the resulting dry powder (hereinafter, this step is referred to as pre-calcination), and a step of subsequent shaping. In this regard, it is also possible to provide a calcination step (main calcination) after the shaping step. Moreover, in the invention, the compound containing active ingredients in the preparation of the slurry not necessarily contains all the active ingredients and a part of ingredients may be added after the drying or after the pre-calcination.

In the catalyst, the kind of the other active ingredients to be used according to needs and the use ratio thereof are appropriately determined in accordance with use conditions and the like of the catalyst or so that a catalyst exhibiting most suitable performance is obtained. As the Y ingredient, cesium is preferably used.

As raw materials for the catalyst, usually, compounds containing individual elements contained in the catalyst, for example, oxo acids, oxo acid salts, oxides, nitrate salts, carbonate salts, hydroxides, halides, and the like of individual elements are used in ratios that satisfy desired atomic ratios. For example, as compounds containing phosphorus, phosphoric acid, phosphate salts, and the like are used; as compounds containing molybdenum, molybdic acid, molybdate salts, molybdenum oxide, molybdenum chloride, and the like are used; as compounds containing vanadium, vanadic acid, vanadate salts, vanadium oxide, vanadium chloride, and the like are used; and, as compounds containing copper, copper nitrate, copper acetate, copper sulfate, copper chloride, copper oxide, and the like are used. In addition, as compounds containing the Y element, oxides, acetate salts, nitrate salts, carbonate salts, hydroxides, halides, and the like are used; and, as compounds containing the Z element, oxo acids, oxo acid salts, nitrate salts, carbonate salts, hydroxides, halides, and the like are used. These compounds containing individual elements contained in the catalyst may be used solely or two or more thereof may be used as a mixture.

The slurry can be obtained by homogeneously mixing individual active ingredient-containing compounds and water. As for the addition order of the active ingredient-containing compounds in the preparation of the slurry, it is preferable that the compounds containing molybdenum, vanadium, phosphorus, and, if necessary, other metal elements are thoroughly dissolved and then cesium-containing compound, an ammonium-containing compound, and a copper-containing compound are added. In the case where an antimony-containing compound is added in the slurry preparation, the compound is preferably added at the last of the essential active ingredient-containing compounds. More preferably, after a slurry containing active ingredients other than the antimony-containing compound is obtained, the slurry is dried and the resulting powder is mixed with the antimony-containing compound, followed by calcination, or the powder is calcined and then mixed with the antimony-containing compound. As for the temperature in the slurry preparation, it is preferable to perform heating to a temperature at which the compounds containing molybdenum, vanadium, phosphorus, and, if necessary, other metal elements can be thoroughly dissolved. Moreover, since the resulting catalyst tends to have high activity when the temperature at the addition of the cesium-containing compound and the ammonium-containing compound is usually in the range of 0 to 35°C, preferably about 10 to 30°C, it is preferable to cool the slurry to 10 to 30°C. The amount of water to be used in the slurry is not particularly limited so long as it is an amount with which the total amount of the compounds to be used can be completely dissolved or can be homogeneously mixed but is appropriately determined in consideration of the drying method, drying conditions, and the like. Usually, based on 100 parts by mass of the total mass of the compounds for slurry preparation, about 200 to 2,000 parts by mass of water is used. The amount of water may be a large amount but when the amount is too large, an energy cost for the drying step increases and there arises a case where the slurry cannot completely be dried.

Then, the slurry obtained in the above is dried to form a dry powder. The drying method is not particularly limited so long as it is a method capable of completely drying the slurry. For example, drum drying, freeze drying, spray drying, evaporation to dryness, and the like may be mentioned. Of these, in the invention, the spray drying capable of drying from a slurry state to a powder or granules for a short period of time is particularly preferred. The drying temperature in spray drying varies depending on the concentration of the slurry, solution-transferring rate, and the like but the outlet temperature of a drier is generally 70 to 150°C. On this occasion, drying is preferably performed so that the average particle size of the slurry-dried material becomes 10 to 700 μm.

Since there is a case where shapability and shape and mechanical strength of a shaped catalyst are remarkably improved by subjecting the resulting dry powder to pre-calcination, pre-calcination is carried out according to needs. The atmosphere for the pre-calcination may be an air stream or a stream of an inert gas such as nitrogen but, industrially, the air stream is preferred. The temperature for the pre-calcination is usually 200 to 400°C, preferably 250 to 380°C, and more preferably 270 to 330°C. Even when the pre-calcination is performed at a temperature lower than 200°C, there is a tendency that influence on shapability decreases. When the temperature exceeds 400°C, the catalyst is prone to be decomposed/sintered, so that performance is sometimes adversely affected. The time for the pre-calcination is preferably 3 to 12 hours and more preferably 5 to 10 hours. The calcination may be performed for 12 hours or more but it is difficult to obtain an effect comparable to such calcination.

Then, the resulting pre-calcined granules are shaped as follows according to needs but shaping is preferably performed after the granules are mixed with a shaping aid such as silica gel, diatomaceous earth or alumina powder because workability is improved. The amount of the shaping aid to be used is usually 1 to 30 parts by mass based on 100 parts by mass of the pre-calcined granules. Moreover, it is useful for enhancing mechanical strength of the catalyst to use further inorganic fibers inactive to the catalyst ingredients, such as ceramic fibers or whiskers, as a strength enhancer according to needs. However, fibers reactive with the catalyst ingredients, such as potassium titanate whiskers or basic magnesium carbonate whiskers, are not preferred. The amount of the fibers to be used is usually 1 to 30 parts by mass based on 100 parts by mass of the pre-calcined granules.

The pre-calcined granules obtained as above or the mixture obtained by mixing the granules with the shaping aid and the strength enhancer is shaped into columnar objects, tablets, rings, spheres, or the like in order to reduce pressure loss of a reactive gas. Of these, since an improvement in selectivity and removal of reaction heat can be expected, it is particularly preferred to coat an inactive support with the pre-calcined granules or the mixture to form a coated catalyst. As a coating step, a rolling granulation method to be described below is preferred. The method is a method of coating a support with the pre-calcined granules or the mixture where, for example, in an apparatus having a flat or uneven disk at the bottom of a fixed container, the support in the container is stirred through repetition of autorotation movement and orbital movement by rotating the disk at a high speed and a binder and the pre-calcined granules or the mixture are added thereto. As methods of adding the binder, methods of 1) mixing the binder with the pre-calcined granules or the mixture beforehand, 2) adding the binder simultaneously when the pre-calcined granules or the mixture are added into the fixed container, 3) adding the binder after the pre-calcined granules or the mixture is added into the fixed container, 4) adding the binder before the pre-calcined granules or the mixture are added into the fixed container, 5) dividing each of the pre-calcined granules or the mixture and the binder and adding all amount of them with appropriately combining 2) to 4), and the like may be arbitrarily adopted. Of these, in the method 5), it is preferred to perform the method with regulating addition rates using an auto feeder or the like so that a predetermined amout is supported on the support without attachment of the pre-calcined granules or the mixture to the wall of the fixed container and aggregation of the pre-calcined granules themselves or the mixture itself.

The binder is preferably at least one selected from the group consisting of water and organic compounds having a boiling point of 150°C or lower under 1 atm and, when drying after coating and the like are considered, an organic compound having a boiling point of 150°C or lower is preferred. Specific examples of the binder other than water include alcohols such as methanol, ethanol, propanols and butanols, preferably alcohols having 1 to 4 carbon atoms, ethers such as ethyl ether, butyl ether and dioxane, esters such as ethyl acetate and butyl acetate, ketones such as acetone and methyl ethyl ketone, aqueous solutions thereof, and the like but ethanol is particularly preferred. In the case where ethanol is used as the binder, ethanol/water is preferably 10/0 to 0/10 (mass ratio) and more preferably 10/0 to 1/9 (mass ratio). The amount of these binders to be used is usually 10 to 60 parts by mass and preferably 15 to 40 parts by mass based on 100 parts by mass of the dry powder.

Specific examples of the support usable in the invention include spherical supports having a diameter of 1 to 15 mm, preferably 2.5 to 10 mm, such as silicon carbide, alumina, silica-alumina, mullite, and alundum. As these supports, those having a porosity of 10 to 70% are usually employed. The ratio of the support to the pre-calcined granules or the mixture to coat is used so that pre-calcined granules or mixture/(pre-calcined granules or mixture + support) becomes usually 10 to 75% by mass and preferably 15 to 60% by mass. Thus, the support is coated with the pre-calcined granules or the mixture and the coated article obtained on this occasion usually has a diameter of about 3 to 15 mm.

The coated catalyst obtained as described above can be provided for the vapor-phase catalytic oxidation reaction as a catalyst without further treatment but calcination is preferably performed since there is a case where the catalytic activity is enhanced. The calcination method and calcination conditions are not particularly limited and known treating methods and conditions can be applied. The most suitable conditions for calcination vary depending on raw materials for the catalyst, catalyst composition, prepartion method, and the like to be used but the temperature is usually 100 to 450°C, preferably 270 to 420°C and the calcination time is 1 to 20 hours. Calcination is usually performed under an air atmosphere but may be performed under an atmosphere of an inert gas such as nitrogen, carbon dioxide, helium or argon or, after calcination under an inert gas atmosphere, calcination may be further performed under an air atmosphere according to needs. The catalyst obtained as above (hereinafter referred to as a catalyst of the invention) is used in the production of methacrylic acid by vapor-phase catalytic oxidation of methacrolein, isobutyraldehyde or isobutyric acid.

The catalyst for methacrylic acid production of the invention as above becomes a catalyst capable of producing methacrylic acid by vapor-phase catalytic oxidation of methacrolein with molecular oxygen in high yields and a long life. As a reason therefor, it is surmised that $\alpha$ influences selectivity of methacrylic acid, the atomic ratio of the Y ingredient influences moisture absorbability of the catalyst as mentioned above and, in the case where $\alpha$ falls within the above range, a chemical structure effective for the reaction of producing methacrylic acid is obtained. The smaller the value of $\alpha$ is, the more the selectivity to methacrylic acid is improved. However, when the atomic ratio of the Y ingredient is smaller than 0.2, most of the counter cations of the hetero polyacid are protons and thus moisture absorbability becomes high.

The following will describe the vapor-phase catalytic reaction using methacrolein that is the most preferable raw material for the use of the catalyst obtained in the invention. For the vapor-phase catalytic oxidation reaction, molecular oxygen or a gas containing molecular oxygen is used. The ratio of the molecular oxygen to be used to methacrolein is preferably the range of 0.5 to 20 and particularly preferably thea range of 1 to 10 as a molar ratio. For the purpose of smooth proceeding of the reaction, it is preferred to add water into a raw material gas in the range of 1 to 20 as a molar ratio. The raw material gas may contain an inert gas inactive to the reaction, such as nitrogen, carbon dioxide or a saturated hydrocarbon, according to needs, other than oxygen and, if necessary, water (usually contained as water vapor). Moreover, methacrolein may be supplied as a gas obtained in the oxidation of isobutylene, tertiary butanol and methyl tertiary butyl ether without further treatment The reaction temperature in the vapor-phase catalytic oxidation reaction is usually 200 to 400°C and preferably 250 to 360°C and the supplying amount of the raw material gas is usually 100 to 6,000 hr$^{-1}$ and preferably 300 to 3,000 hr$^{-1}$ as a space velocity (SV). Furthermore, the catalytic oxidation reaction can be carried out under pressurization or under reduced pressure but generally, a pressure around atmospheric pressure is suitable.

Examples

The following will describe the present invention in further detail with reference to Examples but the invention should not be construed as being limited to Examples unless it exceeds the gist thereof.

In the following, conversion and yield are defined as mentioned below.

$$\text{Conversion of methacrolein} = (\text{Number of moles of supplied methacrolein} - \text{Number of moles of unreacted methacrolein})/\text{Number of moles of supplied methacrolein} \times 100$$

$$\text{Yield of methacrylic acid} = (\text{Number of moles of formed methacrylic acid} - \text{Number of moles of supplied methacrolein})/\text{Number of moles of supplied methacrolein} \times 100$$

$$\text{Selectivity of methacrylic acid} = (\text{Number of moles of formed methacrylic acid} - \text{Number of moles of supplied methacrylic acid})/(\text{Number of moles of supplied methacrolein} - \text{Number of moles of unreacted methacrolein}) \times 100$$

Example 1

1) Preparation of Catalyst

Into 5,680 ml of pure water were charged 800 g of molybdenum trioxide, 40.43 g of vanadium pentoxide, and 73.67 g of an 85% by mass orthophosphoric acid, and the whole was heated and stirred at 92°C for 3 hours to obtain a redish brown transparent solution. Subsequently, the solution was cooled to 15 to 20°C, then 307.9 g of a 9.1% by mass aqueous cesium hydroxide solution and 689.0 g of a 14.3% by mass aqueous ammonium acetate solution were gradually added thereto under stirring, and the whole was aged at 15 to 20°C for 1 hour to obtain a yellow slurry. Then, 709.9 g of a 6.3% by mass aqueous cupric acetate solution was gradually added to the slurry, followed by ageing at 15 to 20°C for 30 minutes. Subsequently, the slurry was spray-dried to obtain granules. The composition of the obtained granules was $Mo_{10}V_{0.8}P_{1.15}Cu_{0.4}Cs_{0.3}(NH_4)_{2.3}$. Then, 320 g of the granules was calcined at 310°C for 5 hours under an air stream to obtain pre-calcined granules. There was a mass decrease of about 4% by mass by the pre-calcination. Therewith were homogeneously mixed 22.7 g of antimony trioxide and 45 g of a strength enhancer (ceramic fibers), and the mixture was coat-shaped on 300 g of a spherical porous alumina support (average particle size: 3.5 mm) using a 20% by mass aqueous ethanol solution as a binder, by a rolling granulation method. Thereafter, the resulting shaped article was subjected to main calcination at 380°C for 5 hours under an air stream to obtain an objective coated catalyst. The composition of the resulting catalyst is $Mo_{10}V_{0.8}P_{1.15}Cu_{0.4}Cs_{0.3}(NH_4)_{2.3}Sb_{1.0}$. Moreover, at this time, $\alpha$ is 1.1.

2) Partial Oxidation Reaction of Methacrolein

Into a stainless steel reaction tube having an inner diameter of 18.4 mm was packed 10.3 ml of the resulting coated catalyst, and an oxidation reaction of methacrolein was carried out at a raw material gas composition (molar ratio) of methacrolein:oxygen:water vapor:nitrogen = 1:2:4:18.6, a space velocity (SV) of 1,200 hr$^{-1}$, and a reaction bath temperature of 310°C. The reaction was first continued at a reaction bath temperature of 310°C for 3 hours, then the reaction bath temperature was elevated to 350°C, and the reaction was continued for 15 hours (hereinafter, the treatment is referred to as high-temperature reaction treatment). Then, the reaction bath temperature was lowered to 310°C and measurement of reaction performance was conducted. Results are shown in Table 1.

Measurement of Moisture Absorption Rate

The resulting coated catalyst was charged into a Petri dish in an amount of 100 g and was allowed to stand for 24 hours in a desiccator that was made saturated vapor pressure at 25°C. Thereafter, the mass of the coated catalyst was measured and was found to be 102.39 g. Namely, the ratio of water absorbed was 5.20% based on the catalyst active ingredient and water absorbed by 100 g of the catalyst active ingredient in dry mass per unit hour was 0.22 g/h. Hereinafter, the value is expressed as a moisture absorption rate and is designated in Table 2.

The catalyst after the measurement of moisture absorption rate was dried in a drier at 120°C for 24 hours. Into a stainless steel reaction tube having an inner diameter of 18.4 mm was packed 10.3 ml of the resulting coated catalyst, and an oxidation reaction of methacrolein was carried out at a raw material gas composition (molar ratio) of methacrolein:oxygen:water vapor:nitrogen = 1:2:4:18.6, a space velocity (SV) of 1,200 hr$^{-1}$, and a reaction bath temperature of 310°C. The reaction was first continued at a reaction bath temperature of 310°C for 3 hours, then the reaction bath temperature was elevated to 350°C, and the reaction was continued for 15 hours (hereinafter, the treatment is referred to as high-temperature reaction treatment). Then, the reaction bath temperature was lowered to 310°C and measurement of reaction performance was conducted. Results are shown in Table 1.

Example 2

A coated catalyst was prepared in the same manner as in Example 1 except that the pre-calcination temperature was changed to 290°C in Example 1. The composition of the obtained catalyst was $Mo_{10}V_{0.8}P_{1.15}Cu_{0.4}Cs_{0.3}(NH_4)_{2.3}Sb_{1.0}$. The oxidation reaction of methacrolein and the measurement of moisture absorption rate were conducted as in Example 1 except that this coated catalyst was used. Results are shown in Tables 1 and 2.

Example 3

A coated catalyst was prepared in the same manner as in Example 1 except that 320 g of the pre-calcined granules, 11.35 g of antimony trioxide, and 45 g of a strength enhancer (ceramic fibers) were homogeneously mixed in Example 1. The composition of the obtained catalyst was $Mo_{10}V_{0.8}P_{1.15}Cu_{0.4}Cs_{0.3}(NH_4)_{2.3}Sb_{0.5}$. Moreover, at this time, $\alpha$ is 1.1. The oxidation reaction of methacrolein and the measurement of moisture absorption rate were conducted as in Example 1 except that this coated catalyst was used. Results are shown in Tables 1 and 2.

Example 4

A coated catalyst was prepared in the same manner as in Example 1 except that 320 g of the pre-calcined granules, 40.9 g of antimony trioxide, and 45 g of a strength enhancer (ceramic fibers) were homogeneously mixed in Example 1. The composition of the obtained catalyst was $Mo_{10}V_{0.8}P_{1.15}Cu_{0.4}Cs_{0.3}(NH_4)_{2.3}Sb_{1.8}$. Moreover, at this time, $\alpha$ is 1.1. The oxidation reaction of methacrolein and the measurement of moisture absorption rate were conducted as in Example 1 except that this coated catalyst was used. Results are shown in Tables 1 and 2.

Example 5

Into 5,680 ml of pure water were charged 800 g of molybdenum trioxide, 30.33 g of vanadium pentoxide and 73.67 g of an 85% by mass orthophosphoric acid, and the whole was heated and stirred at 92°C for 3 hours to obtain a redish brown transparent solution. Subsequently, the solution was cooled to 15 to 20°C, then 661.3 g of a 9.1% by mass aqueous cesium hydroxide solution and 689.0 g of a 14.3% by mass aqueous ammonium acetate solution were gradually added thereto under stirring, and the whole was aged at 15 to 20°C for 1 hour to obtain a yellow slurry. Then, 232.9 g of a 9.5% by mass aqueous cupric acetate solution was gradually added to the slurry, followed by ageing at 15 to 20°C for 30 minutes. Subsequently, the slurry was spray-dried to obtain granules. The composition of the obtained granules was $Mo_{10}V_{0.6}P_{1.15}Cu_{0.2}Cs_{0.7}(NH_4)_{2.3}$. Then, 320 g of the granules was calcined at 310°C for 5 hours under an air stream to obtain pre-calcined granules. There was a mass decrease of about 4% by mass by the pre-calcination. Therewith was homogeneously mixed 45 g of a strength enhancer (ceramic fibers), and the mixture was coat-shaped on 300 g of a spherical porous alumina support (average particle size: 3.5 mm) using a 20% by mass aqueous ethanol solution as a binder, by a rolling granulation method. Then, the resulting shaped article was subjected to main calcination at 380°C for 5 hours under an air stream to obtain an objective coated catalyst. The composition of the resulting catalyst is $Mo_{10}V_{0.6}P_{1.15}Cu_{0.2}Cs_{0.7}(NH_4)_{2.3}$. Moreover, at this time, $\alpha$ is 1.1. The oxidation reaction of methacrolein and the measurement of moisture absorption rate were conducted as in Example 1 except that this coated catalyst was used. Results are shown in Tables 1 and 2.

Example 6

A coated catalyst was prepared in the same manner as in Example 5 except that pure water was used as a binder in Example 5. The composition of the obtained catalyst was $Mo_{10}V_{0.6}P_{1.15}Cu_{0.2}Cs_{0.7}(NH_4)_{2.3}$. Moreover, at this time, $\alpha$ is 1.1. The oxidation reaction of methacrolein and the measurement of moisture absorption rate were conducted as in Example 1 except that this coated catalyst was used. Results are shown in Tables 1 and 2.

Example 7

A coated catalyst was prepared in the same manner as in Example 5 except that a 90% by mass aqueous ethanol solution was used as a binder in Example 5. The composition of the obtained catalyst was $Mo_{10}V_{0.6}P_{1.15}Cu_{0.2}Cs_{0.7}(NH_4)_{2.3}$. Moreover, at this time, $\alpha$ is 1.1. The oxidation reaction of methacrolein and the measurement of moisture absorption rate were conducted as in Example 1 except that this coated catalyst was used. Results are shown in Tables 1 and 2.

Example 8

Into 5,680 ml of pure water were charged 800 g of molybdenum trioxide, 30.33 g of vanadium pentoxide and 76.87 g of an 85% by mass orthophosphoric acid, and the whole was heated and stirred at 92°C for 3 hours to obtain a redish brown transparent solution. Subsequently, the solution was cooled to 15 to 20°C, then 321.2 g of a 9.1% by mass aqueous cesium hydroxide solution and 196.86 g of a 50% by mass aqueous ammonium acetate solution were gradually added thereto under stirring, and the whole was aged at 15 to 20°C for 1 hour to obtain a yellow slurry. Then, 22.18 g of cupric acetate was gradually added to the slurry, followed by ageing at 15 to 20°C for 30 minutes. Subsequently, the slurry was spray-dried to obtain granules. The composition of the obtained granules was $Mo_{10}V_{0.6}P_{1.15}Cu_{0.2}Cs_{0.3}(NH_4)_{2.3}$. Moreover, at this time, $\alpha$ is 0.7. The oxidation reaction of methacrolein was conducted as in Example 1 except that this coated catalyst was used. Results are shown in Table 1.

Comparative Example 1

Into 7,100 ml of pure water were charged 1,000 g of molybdenum trioxide, 75.81 g of vanadium pentoxide, 88.08 g of an 85% by mass orthophosphoric acid and 11.05 g of copper oxide, and the whole was heated and stirred at 92°C for 3 hours to obtain a slurry. Subsequently, the slurry was spray-dried to obtain granules. The composition of the obtained granules was $Mo_{10}V_{1.2}P_{1.1}Cu_{0.2}$. Thereafter, 320 g of the granules was homogeneously mixed with 45 g of a strength enhancer (ceramic fibers), and the mixture was coat-shaped on 300 g of a spherical porous alumina support (average particle size: 3.5 mm) using a 90% by mass aqueous ethanol solution as a binder. Then, the resulting shaped article was subjected to main calcination at 310°C for 5 hours under an air stream to obtain an objective coated catalyst. The composition of the resulting catalyst is $Mo_{10}V_{1.2}P_{1.1}Cu_{0.2}$. Moreover, at this time, $\alpha$ is 0.4. The oxidation reaction of methacrolein and the measurement of moisture absorption rate were conducted as in Example 1 except that this coated catalyst was used. Results are shown in Tables 1 and 2.

Comparative Example 2

Into 10,000 ml of pure water were charged 1,000 g of molybdenum trioxide, 37.91 g of vanadium pentoxide, 96.09 g of an 85% by mass orthophosphoric acid, 65.73 g of a 60% by mass aqueous arsenic acid solution, and 22.1 g of cupric oxide, and the whole was stirred at 92°C for 3 hours to obtain a slurry. Subsequently, the slurry was spray-dried to obtain granules. The composition of the obtained granules was $Mo_{10}V_{0.6}P_{1.2}As_{0.4}Cu_{0.4}$. Thereafter, 320 g of the granules was homogeneously mixed with 45 g of a strength enhancer (ceramic fibers), and the mixture was coat-shaped on 300 g of a spherical porous alumina support (average particle size: 3.5 mm) using a 90% by mass aqueous ethanol solution as a binder. Then, the resulting shaped article was subjected to main calcination at 310°C for 5 hours under an air stream to obtain an objective coated catalyst. The composition of the resulting catalyst is $Mo_{10}V_{0.6}P_{1.2}As_{0.4}Cu_{0.4}$. Moreover, at this time, $\alpha$ is 0.8. The oxidation reaction of methacrolein and the measurement of moisture absorption rate were conducted as in Example 1 except that this coated catalyst was used. Results are shown in Tables 1 and 2.

Comparative Example 3

Into 5,680 ml of pure water were charged 800 g of molybdenum trioxide, 35.38 g of vanadium pentoxide, and 73.67 g of an 85% by mass orthophosphoric acid, and the whole was heated and stirred at 92°C for 3 hours to obtain a redish brown transparent solution. Subsequently, the solution was cooled to 15 to 20°C, then 94.49 g of a 9.1% by mass aqueous cesium hydroxide solution and 988.6 g of a 14.3% by mass aqueous ammonium acetate solution were gradually added thereto under stirring, and the whole was aged at 15 to 20°C for 1 hour to obtain a yellow slurry. Then, 465.9 g of a 6.3% by mass aqueous cupric acetate solution was gradually added to the slurry, followed by ageing at 15 to 20°C for 30 minutes. Subsequently, the slurry was spray-dried to obtain granules. The composition of the obtained granules was $Mo_{10}V_{0.7}P_{1.15}Cu_{0.4}Cs_{0.1}(NH_4)_{2.3}$. Then, 320 g of the granules was calcined at 310°C for 5 hours under an air stream to obtain pre-calcined granules. There was a mass decrease of about 4% by mass by the pre-calcination.

Therewith was homogeneously mixed 45 g of a strength enhancer (ceramic fibers), and the mixture was coat-shaped on 300 g of a spherical porous alumina support (average particle size: 3.5 mm) using a 20% by mass aqueous ethanol solution as a binder, by a rolling granulation method. Then, the resulting shaped article was subjected to main calcination at 380°C for 5 hours under an air stream to obtain an objective coated catalyst. The composition of the resulting catalyst is $Mo_{10}V_{0.7}P_{1.15}Cu_{0.4}Cs_{0.1}(NH_4)_{3.3}$. Moreover, at this time, $\alpha$ is 0.9. The oxidation reaction of methacrolein and the measurement of moisture absorption rate were conducted as in Example 1 except that this coated catalyst was used. Results are shown in Tables 1 and 2.

Comparative Example 4

Into 5,680 ml of pure water were charged 800 g of molybdenum trioxide, 35.38 g of vanadium pentoxide and 73.67 g of an 85% by mass orthophosphoric acid, and the whole was heated and stirred at 92°C for 3 hours to obtain a redish brown transparent solution. Subsequently, the solution was cooled to 15 to 20°C, then 850.32 g of a 9.1% by mass aqueous cesium hydroxide solution and 689.0 g of a 14.3% by mass aqueous ammonium acetate solution were gradually added thereto under stirring, and the whole was aged at 15 to 20°C for 1 hour to obtain a yellow slurry. Then, 233.6 g of a 9.5% by mass aqueous cupric acetate solution was gradually added to the slurry, followed by ageing at 15 to 20°C for 30 minutes. Subsequently, the slurry was spray-dried to obtain granules. The composition of the obtained granules was $Mo_{10}V_{0.7}P_{1.15}Cu_{0.2}Cs_{1.1}(NH_4)_{2.3}$. Then, 320 g of the granules was calcined at 310°C for 5 hours under an air stream to obtain pre-calcined granules. There was a mass decrease of about 4% by mass by the pre-calcination. Therewith was homogeneously mixed 45 g of a strength enhancer (ceramic fibers), and the mixture was coat-shaped on 300 g of a spherical porous alumina support (average particle size: 3.5 mm) using a 20% by mass aqueous ethanol solution as a binder, by a rolling granulation method. Then, the resulting shaped article was subjected to main calcination at 380°C for 5 hours under an air stream to obtain an objective coated catalyst. The composition of the resulting catalyst is $Mo_{10}V_{0.7}P_{1.15}Cu_{0.2}Cs_{1.1}(NH_4)_{2.3}$. Moreover, at this time, $\alpha$ is 1.5. The oxidation reaction of methacrolein and the measurement of moisture absorption rate were conducted as in Example 1 except that this coated catalyst was used. Results are shown in Tables 1 and 2.

Comparative Example 5

Into 5,680 ml of pure water were charged 800 g of molybdenum trioxide, 30.33 g of vanadium pentoxide and 76.87 g of an 85% by mass orthophosphoric acid, and the whole was heated and stirred at 92°C for 3 hours to obtain a redish brown transparent solution. Subsequently, the solution was cooled to 15 to 20°C, then 94.5 g of a 9.1% by mass aqueous cesium hydroxide solution and 196.86 g of a 50% by mass aqueous ammonium acetate solution were gradually added thereto under stirring, and the whole was aged at 15 to 20°C for 1 hour to obtain a yellow slurry. Then, 11.09 g of cupric acetate was gradually added to the slurry, followed by ageing at 15 to 20°C for 30 minutes. Subsequently, the slurry was spray-dried to obtain granules. The composition of the obtained granules was $Mo_{10}V_{0.6}P_{1.2}Cu_{0.1}Cs_{0.1}(NH_4)_{2.3}$. Moreover, at this time, $\alpha$ is 0.3. The oxidation reaction of methacrolein was conducted as in Example 1 except that this coated catalyst was used. Results are shown in Tables 1 and 2.

Comparative Example 6

Into 5,680 ml of pure water were charged 800 g of molybdenum trioxide, 40.43 g of vanadium pentoxide and 73.67 g of an 85% by mass orthophosphoric acid, and the whole was heated and stirred at 92°C for 3 hours to obtain a redish brown transparent solution. Subsequently, the solution was cooled to 15 to 20°C, then 944.8 g of a 9.1% by mass aqueous cesium hydroxide solution and 205.42 g of a 50% by mass aqueous ammonium acetate solution were gradually added thereto under stirring, and the whole was aged at 15 to 20°C for 1 hour to obtain a yellow slurry. Then, 44.37 g of cupric acetate was gradually added to the slurry, followed by ageing at 15 to 20°C for 30 minutes. Subsequently, the slurry was spray-dried to obtain granules. The composition of the obtained granules was $Mo_{10}V_{0.8}P_{1.15}Cu_{0.4}Cs_{1.0}(NH_4)_{2.4}$. Then, 320 g of the granules was calcined at 310°C for 5 hours under an air stream to obtain pre-calcined granules. There was a mass decrease of about 4% by mass by the pre-calcination. Therewith were homogeneously mixed 21.0 g of antimony trioxide and 45 g of a strength enhancer (ceramic fibers), and the mixture was coat-shaped on 300 g of a spherical porous alumina support (average particle size: 3.5 mm) using a 20% by mass aqueous ethanol solution as a binder, by a rolling granulation method. Then, the resulting shaped article was subjected to main calcination at 380°C for 5 hours under an air stream to obtain an objective coated catalyst. The composition of the resulting catalyst is $Mo_{10}V_{0.8}P_{1.15}Cu_{0.4}Cs_{1.0}(NH_4)_{2.4}Sb_{1.0}$. Moreover, at this time, $\alpha$ is 1.8. The oxidation reaction of methacrolein was conducted as in Example 1 except that this coated catalyst was used. Results are shown in Table 1.

[Table 1]

Table 1

| | | | Conversion of methacrolein (%) | Selectivity of methacrylic acid (%) | Yield of methacrylic acid (%) |
|---|---|---|---|---|---|
| Example 1 | No moisture absorption | Initial stage of reaction | 90.31 | 80.50 | 72.70 |
| | | After high-temperature reaction treatment | 92.08 | 80.58 | 74.20 |
| | After 24h moisture absorption | Initial stage of reaction | 88.57 | 82.35 | 72.94 |
| | | After high-temperature reaction treatment | 91.34 | 82.91 | 75.73 |
| Example 2 | No moisture absorption | Initial stage of reaction | 82.44 | 79.97 | 65.93 |
| | | After high-temperature reaction treatment | 88.25 | 82.32 | 72.65 |
| | After 24h moisture absorption | Initial stage or reaction | 80.21 | 84.00 | 67.38 |
| | | After high-temperature reaction treatment | 86.66 | 85.10 | 73.75 |
| Example 3 | No moisture absorption | Initial stage of reaction | 81.71 | 81.16 | 66.32 |
| | | After high-temperature reaction treatment | 87.82 | 83.61 | 73.42 |
| | After 24h moisture absorption | Initial stage of reaction | 79.08 | 86.65 | 66.15 |
| | | After high-temperature reaction treatment | 83.01 | 88.34 | 73.33 |
| Example 4 | No moisture absorption | Initial stage of reaction | 87.29 | 81.19 | 70.85 |
| | | After high-temperature reaction treatment | 89.69 | 83.50 | 74.89 |
| | After 24h moisture absorption | Initial stage of reaction | 83.30 | 78.68 | 65.54 |
| | | After high-temperature reaction treatment | 87.66 | 81.58 | 71.52 |
| Example 5 | No moisture absorption | Initial stage of reaction | 92.35 | 7812 | 72.15 |
| | | After high-temperature reaction treatment | 93.17 | 79.92 | 74.45 |
| | After 24h moisture absorption | Initial stage of reaction | 89.10 | 80.05 | 71.32 |

(continued)

|  |  |  | Conversion of methacrolein (%) | Selectivity of methacrylic acid (%) | Yield of methacrylic acid (%) |
|---|---|---|---|---|---|
|  |  | After high-temperature reaction treatment | 91.46 | 81.73 | 74.75 |
| Example 6 | No moisture absorption | Initial stage of reaction | 93.32 | 79.57 | 74.26 |
|  |  | After high-temperature reaction treatment | 92.27 | 83.22 | 76.79 |
|  | After 24h moisture absorption | Initial stage of reaction | 92.27 | 79.83 | 73.66 |
|  |  | After high-temperature reaction treatment | 91.74 | 82.07 | 75.29 |
| Example 7 | No moisture absorption | Initial stage of reaction | 91.53 | 82.24 | 75.27 |
|  |  | After high temperature reaction treatment | 92.93 | 82.25 | 76.43 |
|  | After 24h moisture absorption | Initial stage of reaction | 91.46 | 80.05 | 73 21 |
|  |  | After high-temperature reaction treatment | 92.33 | 81.52 | 75.27 |
| Example 8 | No moisture absorption | Initial stage of reaction | 83.38 | 82.51 | 68.80 |
|  |  | After high-temperature reaction treatment | 92.06 | 83.57 | 76.93 |

[Table 2]

| Table 1 (continued) | | | | | |
|---|---|---|---|---|---|
|  |  |  | Conversion of methacrolein (%) | Selectivity of methacrylic acid (%) | Yield of methacrylic acid (%) |
| Comparative Example 1 | No moisture absorption | Initial stage of reaction | 63.50 | 79.17 | 50.28 |
|  |  | After high-temperature reaction treatment | 74.96 | 80.62 | 60.43 |
|  | After 24h moisture absorption | Initial stage of reaction | 14.88 | 80.93 | 12.04 |
|  |  | After high-temperature reaction treatment | 15.37 | 81.74 | 12.56 |
| Comparative Example 2 | No moisture absorption | Initial stage of reaction | 76.63 | 79.74 | 61.10 |

13

(continued)

| Table 1 (continued) | | | | | |
|---|---|---|---|---|---|
| | | | Conversion of methacrolein (%) | Selectivity of methacrylic acid (%) | Yield of methacrylic acid (%) |
| | | After high-temperature reaction treatment | 82.39 | 79.72 | 65.68 |
| | After 24h moisture absorption | Initial stage of reaction | 18.44 | 80.98 | 14.93 |
| | | After high-temperature reaction treatment | 19.18 | 8168 | 15.66 |
| Comparative Example 3 | No moisture absorption | Initial stage of reaction | 71.89 | 77.86 | 55.97 |
| | | After high-temperature reaction treatment | 79.03 | 83.24 | 65.78 |
| | After 24h moisture absorption | Initial stage of reaction | 71.26 | 86.66 | 61.75 |
| | | After high-temperature reaction treatment | 70.57 | 88.57 | 62.50 |
| Comparative Example 4 | No moisture absorption | Initial stage of reaction | 67.23 | 85.62 | 57.56 |
| | | After high-temperature reaction treatment | 79.98 | 86.27 | 69.00 |
| | After 24h moisture absorption | Initial stage of reaction | 42.02 | 88.45 | 37.17 |
| | | After high-temperature reaction treatment | 48.57 | 90.11 | 43.77 |

| Table 1 (continued) | | | | | |
|---|---|---|---|---|---|
| | | | Conversion of methacrolein (%) | Selectivity of methacrylic acid (%) | Yield of methacrylic acid (%) |
| Comparative Example 5 | No moisture absorption | Initial stage of reaction | 63.02 | 86.99 | 54.82 |
| | | After high-temperature reaction treatment | 76.14 | 87.96 | 66.98 |
| | After 24h moisture absorption | Initial stage of reaction | 58.42 | 87.66 | 51.21 |
| | | After high-temperature reaction treatment | 73.91 | 89.35 | 66.04 |
| Comparative Example 6 | No moisture absorption | Initial stage of reaction | 80.50 | 81.50 | 65.61 |

(continued)

| Table 1 (continued) | | | | | |
|---|---|---|---|---|---|
| | | | Conversion of methacrolein (%) | Selectivity of methacrylic acid (%) | Yield of methacrylic acid (%) |
| | | After high-temperature reaction treatment | 72.00 | 85.50 | 61.56 |

[Table 3]

Table 2

| | Moisture absorption rate (g/h) |
|---|---|
| Example 1 | 0.22 |
| Example 2 | 0.26 |
| Example 3 | 0.30 |
| Example 4 | 0.28 |
| Example 5 | 0.24 |
| Example 6 | 0.22 |
| Example 7 | 0.21 |
| Comparative Example 1 | 0.63 |
| Comparative Example 2 | 0.70 |
| Comparative Example 3 | 0.35 |
| Comparative Example 4 | 0.41 |
| Comparative Example 5 | 0.88 |

Test Example 1

Into a stainless steel reaction tube having an inner diameter of 18.4 mm was packed 6.9 ml of the coated catalyst obtained in Example 1, and supply was performed so as to be a raw material gas composition (molar ratio) of methacrolein:oxygen:water vapor.nitrogen =1:2:4:18.6 and a space velocity (SV) of 1,800 hr$^{-1}$. After start of the reaction, the partial oxidation reaction of methacrolein was continued with controlling the reaction bath temperature so that conversion of methacrolein became 75%$\pm$2%. Results of the oxidation reaction of methacrolein after 800 hours from the start of the reaction were as follows: reaction bath temperature = 344°C, hot spot temperature = 355°C, conversion of methacrolein = 75.5%, yield of methacrylic acid = 62.3%, and selectivity of methacrylic acid = 82.7%.

Test Example 2

Into a stainless steel reaction tube having an inner diameter of 18.4 mm was packed 6.9 ml of the coated catalyst obtained in Example 5, and supply was performed so as to be a raw material gas composition (molar ratio) of methacrolein:oxygen:water vapor:nitrogen = 1:2:4:18.6 and a space velocity (SV) of 1,800 hr$^{-1}$. After start of the reaction, the partial oxidation reaction of methacrolein was continued with controlling the reaction bath temperature so that conversion of methacrolein became 75%$\pm$2%. Results of the oxidation reaction of methacrolein after 800 hours from the start of the reaction were as follows: reaction bath temperature = 325°C, hot spot temperature = 336°C, conversion of methacrolein = 75.7%, yield of methacrylic acid = 63.4%, and selectivity of methacrylic acid = 83.8%.

Test Example 3

Into a stainless steel reaction tube having an inner diameter of 18.4 mm was packed 6.9 ml of the coated catalyst obtained in Comparative Example 4, and supply was performed so as to be a raw material gas composition (molar ratio) of methacrolein:oxygen:water vapor:nitrogen = 1:2:4:18.6 and a space velocity (SV) of 1,800 hr$^{-1}$. After start of the reaction, the partial oxidation reaction of methacrolein was continued with controlling the reaction bath temperature so that conversion of methacrolein became 75%±2%. Results of the oxidation reaction of methacrolein after 800 hours from the start of the reaction were as follows: reaction bath temperature = 346°C, hot spot temperature = 359°C, conversion of methacrolein = 75.1%, yield of methacrylic acid = 58.7%, and selectivity of methacrylic acid = 78.2%.

While the invention has been described in detail and with reference to specific embodiments thereof, it will be apparent to one skilled in the art that various changes and modifications can be made therein without departing from the spirit and scope thereof.

The present application is based on Japanese Patent Application No. 2011-251386 filed on November 17,2011, and the contents are incorporated herein by reference. Also, all the references cited herein are incorporated as a whole.

Industrial Applicability

According to the invention, in the catalyst used in the production of methacrylic acid, it becomes possible to make it more excellent in performance, life, and moisture absorption during storage that is particularly important in industrial use.

The catalyst of the invention is useful for producing methacrylic acid by vapor-phase catalytic oxidation of at least one selected from the group consisting of methacrolein, isobutyraldehyde and isobutyric acid with a molecular oxygen-containing gas in the presence of an oxidation catalyst composition.

**Claims**

1. A catalyst for methacrylic acid production used for producing methacrylic acid by vapor-phase catalytic oxidation of methacrolein with molecular oxygen,
the catalyst having a composition represented by the following general formula:

$$Mo_aP_bV_cCu_dY_eZ_fO_g$$

wherein Mo, P, V, Cu and O represent molybdenum, phosphorus, vanadium, copper and oxygen, respectively; Y represents at least one element selected from potassium, rubidium, cesium and thallium; Z represents at least one element selected from iron, cobalt, zinc, chromium, magnesium, tantalum, manganese, gallium, barium, cerium, lanthanum, arsenic, antimony, bismuth, germanium, ammonium, zirconium, tin, lead, titanium, tellurium, silver, selenium, silicon, tungsten and boron; a, b, c, d, e, f and g represent each an atomic ratio of each element and, when a is 10, b is 0.1 or more and 4 or less, c is 0.01 or more and 4 or less, d is 0.01 or more and 1 or less, e is 0.2 or more and 2 or less, f is 0 or more and 3 or less, and g is a numerical value determined depending on oxidation states of individual elements,
wherein a proton that is a counter cation is replaced by an alkali metal ion so as to satisfy conditions:

$$\alpha = A + (B \times C)$$

$$0.5 \leq \alpha \leq 1.4$$

when an atomic ratio of the alkali metal atom relative to 10 atoms of molybdenum in a hetero polyacid and hetero polyacid salt containing molybdenum, phosphorus, vanadium and copper as essential ingredients is taken as A and an atomic ratio of copper atom relative to 10 atoms of molybdenum in the hetero polyacid and hetero polyacid salt is taken as B, and a valence number is taken as C.

2. The catalyst for methacrylic acid production according to claim 1, which satisfies a condition:

$$0.7 \leq \alpha \leq 1.1.$$

3. The catalyst for methacrylic acid production according to claim 1 or 2,

wherein Y is cesium.

4. The catalyst for methacrylic acid production according to any one of claims 1 to 3, which satisfies conditions that d is 0.1 or more and 0.3 or less and e is 0.3 or more and 1.1 or less when a is 10.

5. The catalyst for methacrylic acid production according to any one of claims 1 to 4, which satisfies conditions that d is 0.15 or more and 0.25 or less and e is 0.4 or more and 1.0 or less when a is 10.

6. The catalyst for methacrylic acid production according to any one of claims 1 to 5, wherein the catalyst is a shaped catalyst.

7. A process for producing methacrylic acid by vapor-phase catalytic oxidation of methacrolein, isobutyraldehyde and isobutyric acid, the process comprising using the catalyst according to any one of claims 1 to 6.

## INTERNATIONAL SEARCH REPORT

| International application No. |
| --- |
| PCT/JP2012/079862 |

### A. CLASSIFICATION OF SUBJECT MATTER
*B01J27/199*(2006.01)i, *C07C51/235*(2006.01)i, *C07C57/055*(2006.01)i, *C07B61/00*(2006.01)n

According to International Patent Classification (IPC) or to both national classification and IPC

### B. FIELDS SEARCHED

Minimum documentation searched (classification system followed by classification symbols)
B01J27/199, C07C51/235, C07C57/055, C07B61/00

Documentation searched other than minimum documentation to the extent that such documents are included in the fields searched
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2011-173114 A (Mitsubishi Rayon Co., Ltd.), 08 September 2011 (08.09.2011), paragraphs [0057] to [0098] (Family: none) | 1-7 |
| X | WO 2011/065529 A1 (Nippon Kayaku Co., Ltd.), 03 June 2011 (03.06.2011), paragraphs [0045] to [0047] & EP 2508256 A1 | 1-7 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 February, 2013 (06.02.13) | 19 February, 2013 (19.02.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60239439 A
- JP 10258233 A
- JP 59066349 A
- JP 2003010695 A
- JP 3884967 B
- JP 5031368 A
- JP 9290162 A
- JP 2011251386 A